# EUROPEAN PATENT APPLICATION

(11) **EP 2 098 993 A1**
(43) Date of publication of application: **09.09.2009**
(21) Application number: 09002352.4
(22) Date of filing: 19.02.2009
(51) Int. Cl.: G06T 7/60, G01S 15/89, A61B 8/08

(54) **Volume measurement in an ultrasound system**

(30) Priority: 05.03.2008 KR 20080020301
(71) Applicant: MEDISON CO., LTD., Kangwon-do 250-870 (KR)
(72) Inventor: Choi, Doo Hyun, Gangnam-gu Seoul 135-851 (KR)
(74) Representative: Schmid, Wolfgang

(57) **Abstract**

Embodiments for measuring volumes of one or more parts of interest in an ultrasound system are disclosed. In one embodiment, the ultrasound system (100) comprises: a volume data forming unit (120) for forming volume data based on ultrasound echoes reflected from a target object, the target object including at least one part of interest (321-323); an interface unit (130) for allowing a user to input a selection instruction for selecting a reference plane (310) from the volume data and a slice setting instruction for setting a plurality of slices (412,414,421-424,431-435) perpendicular to the reference plane (310) in the volume data; an image processing unit (150) for detecting a contour (311-313) of the part of interest on the reference plane and determining intersection points of the detected contour and each of the slices as seed points (321-323a,b), the image processing unit (150) being further configured to detect contours (361,362,530) of the part of interest on the slices based on the seed points; and a volume measuring unit (160) for measuring a volume of the part of interest based on the contour on the reference plane and the contours on the slices.

## Description

The present application claims priority from Korean Patent Application No. 10-2008-0020301 filed on March 5, 2008, the entire subject matter of which is incorporated herein by reference.

### TECHNICAL FIELD

The present disclosure relates to ultrasound systems, and more particularly to volume measurement in an ultrasound system.

### BACKGROUND

Recently, an ultrasound system has been extensively used in the medical field due to its non-invasive and non-destructive nature. The ultrasound system operates in various image modes such as a brightness mode, a Doppler mode and the like to acquire ultrasound images for diagnosis. Also, the ultrasound system may operate in a measure mode for measuring volumes of parts of interest (e.g., organs, lesions, etc.) contained in a target object. Volume data, which are usually acquired in the brightness mode, may be used to measure the volumes of parts of interest.

If the measure mode is set, then the ultrasound system allows a user to input a selection instruction for selecting a reference plane from the volume data. Further, a slice setting instruction for setting a plurality of slices, which are perpendicular to the reference plane and parallel to each other, is inputted. The ultrasound system then sets a seed point at a center of each of the slices to extract a contour of the part of interest on each of the slices. The ultrasound system measures the volume of part of interest based on the extracted contours of the part of interest.

In the conventional ultrasound system, however, only one seed point is set at the center of each slice. As such, an error may occur in extracting the contours of the part of interest on the slices. Thus, the volume of part of interest may be inaccurately calculated. Also, since one seed point is set, a volume for only one part of interest can be measured. That is, when a plurality of parts of interest is contained in the target object, the conventional ultrasound system cannot measure the volumes of parts of interest at the same time.

### SUMMARY

Embodiments for measuring volumes of one or more parts of interest are disclosed herein. In one embodiment, by way of non-limiting example, an ultrasound system comprises a volume data forming unit for forming volume data based on ultrasound echoes reflected from a target object, the target object including at least one part of interest; an interface unit for allowing a user to input a selection instruction for selecting a reference plane from the volume data and a slice setting instruction for setting a plurality of slices perpendicular to the reference plane in the volume data; an image processing unit for detecting a contour of the part of interest on the reference plane and determining intersection points of the detected contour and each of the slices as seed points, the image processing unit being further configured to detect contours of the part of interest on the slices based on the seed points; and a volume measuring unit for measuring a volume of part of interest based on the contour on the reference plane and the contours on the slices.

In another embodiment, a method of measuring a volume of at least one part of interest contained in a target object in an ultrasound system, which includes a volume data forming unit, an interface unit, an image processing unit and a volume measuring unit, comprises the following steps: a) at the volume data forming unit, forming volume data based on ultrasound echoes reflected from a target object containing at least one part of interest; b) at the interface unit, receiving a selection instruction for selecting a reference plane from the volume data; c) at the image processing unit, detecting a contour of the part of interest on the reference plane; d) at the interface unit, receiving a slice setting instruction for setting a plurality of slices perpendicular to the reference plane in the volume data; e) at the image processing unit, determining intersection points of the detected contour and each of the slices as seed points and detecting contours of the part of interest on the slices based on the seed points; and f) at the volume measuring, measuring a volume of the part of interest based on the contour on the reference plane and the contours on the slices.

The Summary is provided to introduce a selection of concepts in a simplified form that are further described below in the Detailed Description. This Summary is not intended to identify key or essential features of the claimed subject matter, nor is it intended to be used in determining the scope of the claimed subject matter.

### BRIEF DESCRIPTION OF THE DRAWINGS

- FIG 1: is a block diagram showing an illustrative embodiment of an ultrasound system.
- FIG 2: is a schematic diagram illustrating volume data.
- FIG 3: is a schematic diagram showing an example of displaying an ultrasound image of a target object containing a plurality of parts of interest.
- FIG. 4: is a block diagram showing an illustrative embodiment of an image processing unit.
- FIG 5: is a schematic diagram showing an example of setting a plurality of slices on a reference plane for determining seed points.
- FIG 6: is a schematic diagram showing an example of detecting a contour of a part of interest on a slice by using seed points and a center point of the seed points.
- FIG. 7: is a schematic diagram showing an example wherein one contour encompasses the other contour.

### DETAILED DESCRIPTION

A detailed description may be provided with reference to the accompanying drawings. One of ordinary skill in the art may realize that the following description is illustrative only and is not in any way limiting. Other embodiments of the present invention may readily suggest themselves to such skilled persons having the benefit of this disclosure.

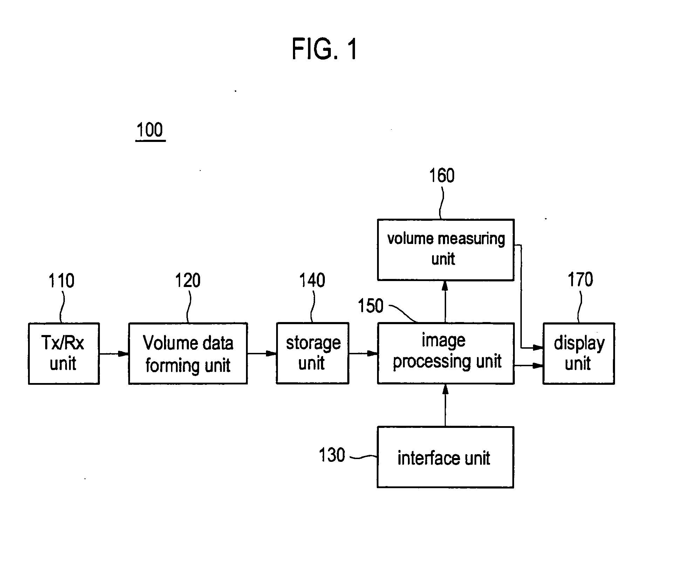

Referring to FIG 1, an illustrative embodiment of an ultrasound system 100 for measuring volumes of parts of interest contained in a target object is shown. As depicted, the ultrasound system 100 may include a transmission/reception (Tx/Rx) unit 110. The Tx/Rx unit 110 may be configured to transmit ultrasound signals to a target object and receive ultrasound echoes reflected from the target object. The Tx/Rx unit 110 may be further configured to convert the ultrasound echoes into electrical signals (hereinafter, referred to as "receive signals"). The Tx/Rx unit 110 may include a probe (not shown) containing a plurality of elements for performing reciprocal conversion of ultrasound signals and electrical signals. The Tx/Rx unit 110 may also include a beamformer (not shown) for performing Tx focusing for the ultrasound signals and Rx focusing for the receive signals.

The ultrasound system 100 may further include a volume data forming unit 120 that may receive the receive signals from the Tx/Rx unit 110. A volume data forming unit 120 may form volume data of the target object containing one or more parts of interest (e.g., organs or lesions) based on the receive signals.

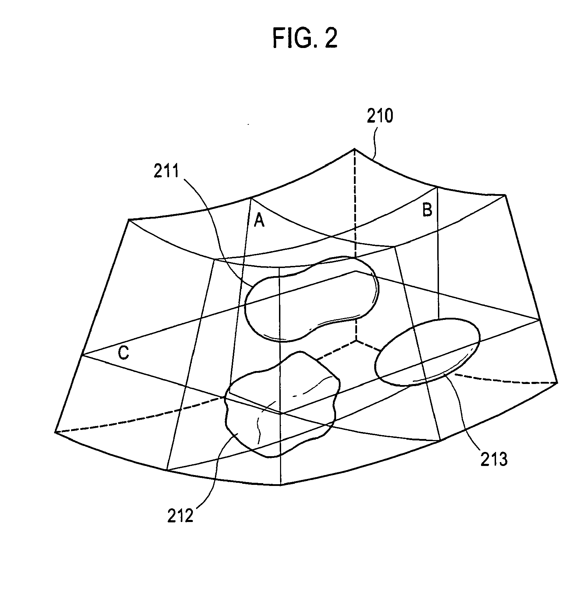

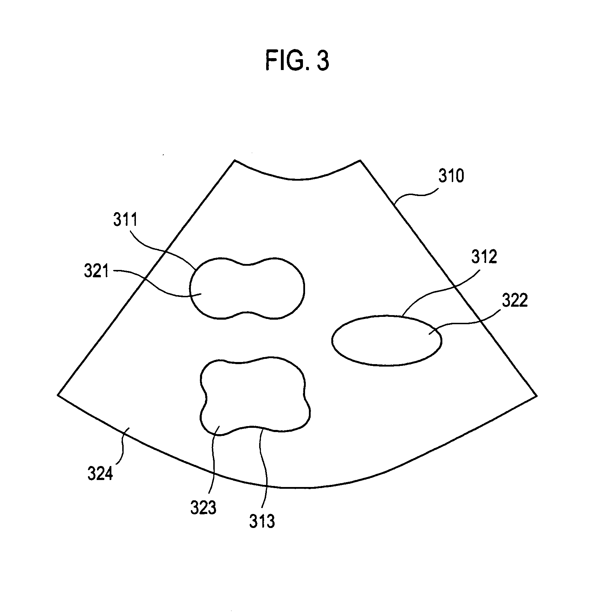

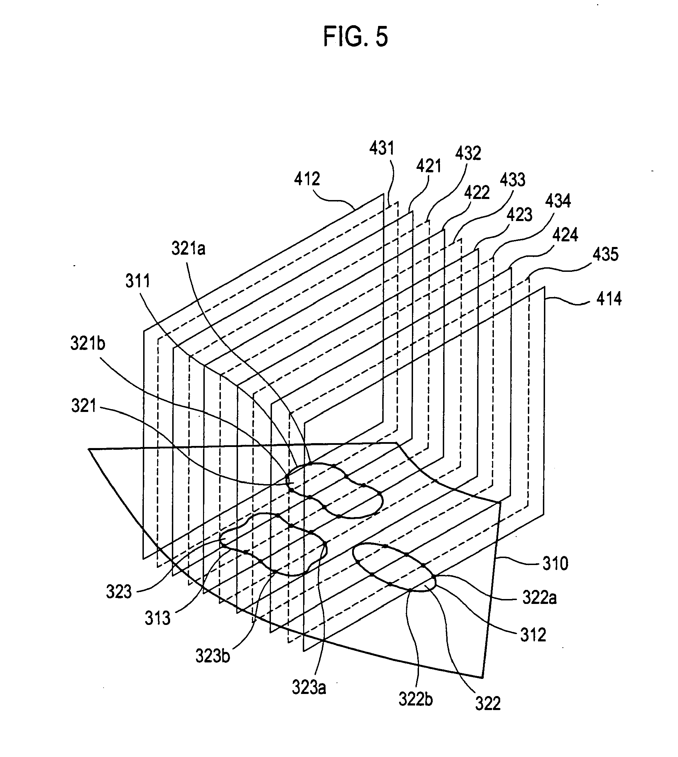

The ultrasound system 100 may further include an interface unit 130 for allowing a user to input instructions. The instructions may include a selection instruction for selecting a reference plane from the volume data, a contour setting instruction for setting contours of the parts of interest and a slice setting instruction for setting slices on the reference plane. Also, an interface unit 130 may include a display unit capable of allowing the user to input the instructions while displaying an ultrasound image. In one embodiment, the reference plane, the contours and the slices may be defined as follows:
(1) The reference plane may be one of an A plane, B plane and C plane in the volume data forming unit 210, as illustrated in FIG 2. However, the reference plane may not be limited thereto. An arbitrary oblique plane for the A, B or C plane may be also set as the reference plane.
(2) The contours may be used to separate a part of interest desired to measure a volume thereof from the other parts on an image of the reference plane (hereinafter, referred to as "reference plane image"). As illustrated in FIG. 3, for example, contours 311, 312 and 313 may separate parts of interest 321, 322 and 323 from the other part 324 on the reference plane image 310.
(3) The slices may be set to be perpendicular to the reference plane. For example, assuming that the A plane is set as a reference plane, the slices may be planes parallel with the B plane (including the B plane), or planes parallel with the C plane (including the C plane). Also, reference slices 412 and 414 may be set on both ends on the reference plane 310 in response to the slice setting instruction, as illustrated in FIG. 5. The number of slices to be set between the reference slices 412 and 414 may be also determined in response to the slice setting instruction. Accuracy for the volume measurement of the parts of interest may depend on the reference slices and the number of slices. The reference slices and the number of slices may be appropriately set according to the experience of the user for accurate volume measurement.

The ultrasound system 100 may further include a storage unit 140 that may store the volume data formed in the volume data forming unit 120. The storage unit 140 may further store instructions to implement a contour setting algorithm for automatically setting contours of parts of interest on the ultrasound image. The contour setting algorithm may be implemented based on previously collected contour information of the parts of interest such as human organs. In one embodiment, the contours of the parts of interest on the reference plane image may be automatically set.

The ultrasound system 100 may also include an image processing unit 150. The image processing unit 150 may be configured to form the reference plane image in response to the selection instruction. The image processing unit 150 may further operate to detect the contours of the parts of interest on the reference plane image in response to the contour setting instruction. The image processing unit 150 may form images of a plurality of slices selected in response to the slice selecting instruction (hereinafter, referred to as "slice images"). The image processing unit 150 may set the parts of interest on each of the slice images and determine seed points of the parts of interest based on the previously collected contour information and the slice information. The seed point determination will be described later in detail. The image processing unit 150 may be further configured to detect the contours of the parts of interest based on the determined seed points.

The ultrasound system 100 may further include a volume measuring unit 160. The volume measuring unit 160 may be configured to measure volumes of the parts of interest based on the contour detected on the reference plane image and the contours detected on the slice image.

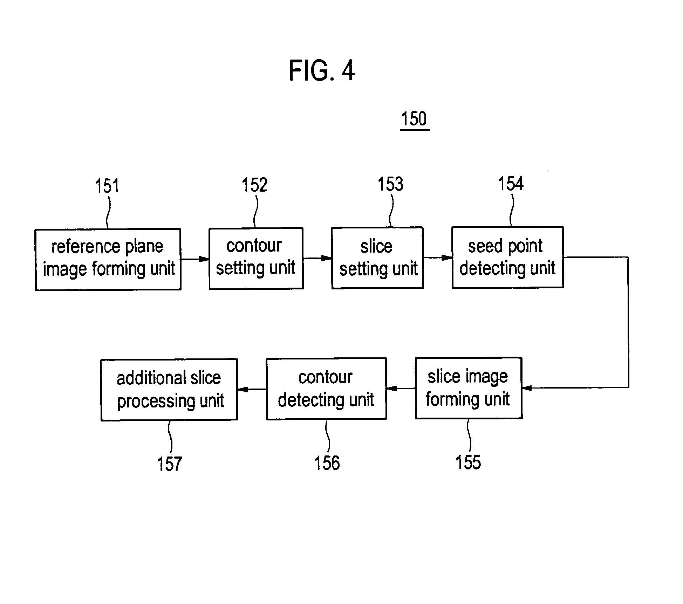

Referring to FIG 4, an illustrative embodiment of the image processing unit 150 is shown. The image processing unit 150 may include a reference plane setting unit 151. The reference plane setting unit 151 may set the reference plane from the volume data in response to the selection instruction. The reference plane setting unit 151 may extract data corresponding to the reference plane from the volume data to form the reference plane image. The ultrasound system may also include a display unit 170 and the reference plane image may be displayed on the display unit 170.

The image processing unit 150 may further include a contour setting unit 152. If the contour setting instruction is inputted through an interface unit 130, then contour setting unit 52 may set contours 311, 312 and 313 of the parts of interest 321, 322 and 323 on the reference plane image 210, as shown in FIG 3. On the other hand, if the contour setting instruction is not inputted for a predetermined time period, then the contour setting unit 152 may set the contours of the parts of interest on the reference plane image 310 based on previously set contour information, which may be previously stored in the storage unit 140.

The image processing unit 150 may further include a slice setting unit 153. If the slice setting instruction is inputted through the interface unit 130, then the slice setting unit 153 may set a plurality of slices on the reference plane image 210. The slice setting instruction may include information upon reference slices and the number of slices to be inserted between first and second reference slices 412 and 414. In one embodiment, the slice setting unit 153 may set first and second reference slices 412 and 414 on the reference plane image 210 based on the reference slice information, as illustrated in FIG. 5. The slice setting unit 153 may further set a plurality of slices 421, 422, 423 and 424 between the first slice 412 and the second slice 414 based on the slice number information (e.g., the number of slices = 4). The slices 421 to 424 may be set at a constant interval between the first slice 412 and the second slice 414.

The image processing unit 150 may also include a seed point determining unit 154. The seed point determining unit 154 may determine seed points on the slice images based on the contours of the parts of interest set on the reference plane image. In one embodiment, the seed point determining unit 154 may determine two points where the slices and the contour of each of the parts of interest intersect, as illustrated in FIG 5. The seed point determining unit 154 may set the two points 321a and 321b, 322a and 322b, and 323a and 323b as seed points for the parts of interest 321, 322 and 323, respectively.

The image processing unit 150 may further include a slice image forming unit 155.

The slice image forming unit 155 may extract data corresponding to the slices from the volume data stored in the storage unit 140 based on the slices 412, 414 and 421-424, which are set on the reference plane image 210. The slice image forming unit 155 may form slice images corresponding to the slices based on the extracted data. The slice image may be displayed on the display unit 170.

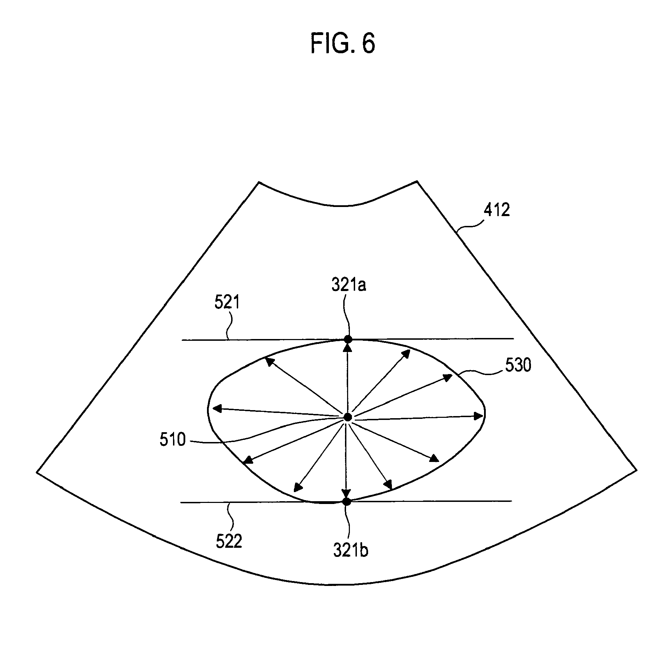

The image processing unit 150 may also include a contour detecting unit 156. The contour detecting unit 156 may detect the contours of the parts of interest on the slice images based on the determined seed points. Referring to FIG 6, the contour detecting unit 156 may set a center point 510 positioned at a center of two seed points 321a and 321b on the slice 412. The contour detecting unit 156 may detect points corresponding to the contour of a part of interest in radial directions with respect to the center point, i.e., edges 530. In one embodiment, the edges 530 of the part of interest may be determined between a line 521 and a line 522, which are defined by two seed points 321a and 321b. The edge detection may be carried out through a conventional method. As such, detailed descriptions thereof are omitted herein. The contour detecting unit 156 may connect the detected edges to each other so that the contour of the part of interest may be set. The contour detecting unit 156 may set the contours of the parts of interest for the entire slices 412, 414 and 421-424 through the same process as described above.

The image processing unit 150 may further include an additional slice processing unit 157. The additional slice processing unit 157 may set additional slices between two reference slices 412 and 414. The additional slice processing unit 157 may detect the contours of the parts of interest from the additional slices by using linear interpolation. In one embodiment, the additional slice processing unit 157 may set an additional slice 431 between the slices 412 and 421. The additional slice processing unit 157 may detect the contours of the parts of interest on the additional slice 431 by performing linear interpolation upon the contours of the parts of interest on the slice 412 and the contours of the parts of interest on the slice 421. The additional slice processing unit 157 may further set additional slices 432-435 in the same manner as described above and then detect the contours of the parts of interest on the additional slices 432-435. The ultrasound system 100 may also include a volume measuring unit 160. The volume measuring unit 160 may measure an area of the reference part of interest based on the contour of the reference part of interest. Also, the volume measuring unit 160 may measure areas of the parts of interest based on the contours of the parts of interest. In one embodiment, a conventional method of measuring the area with the contour may be adopted. Thus, detailed descriptions of the area measurement will be omitted herein. The volume measuring unit 160 may measure a volume of each of the parts of interest based on the area of the reference part of interest and the areas of the parts of interest. In such a case, the measured volume value may be displayed through the display unit 170. The volume value may be represented by numeral texts, a bar graph or the like.

In one embodiment, the image processing unit 150 may form 3-dimensional ultrasound images of the parts of interest based on the contour of the reference part of interest on the reference slice image and the contours of the parts of interest on the slice images. When the 3-dimensional ultrasound images corresponding to the parts of interest are displayed on the display unit 170 at the same time, overlapping portions may occur. Thus, the image processing unit 150 may apply weights to the 3-dimensional images such that the 3-dimensional images have different transparencies.

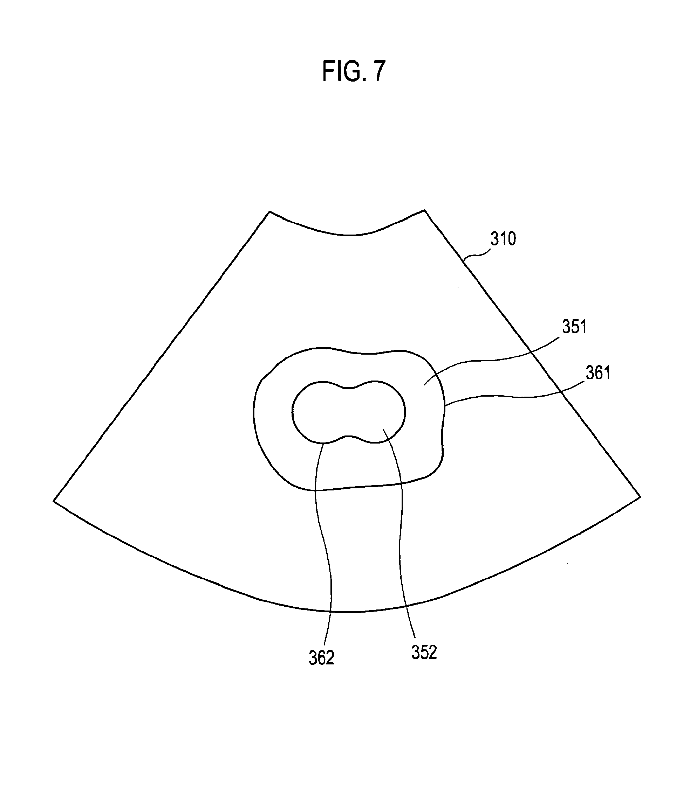

Although it is described that the contours include the contours for setting the parts of interest, which are separate from each other, on the reference plane image as illustrated in FIG 3, the contours are certainly not limited thereto. As illustrated in FIG 7, the contours may include a first contour 361 for separating a first part of interest 351 from the other parts and a second contour 362 for setting a second part of interest 352 within the first part of interest 351.

As mentioned above, the seed points may be automatically and accurately set by using the reference plane image and the slice images. This is so that the contours of the parts of interest on the slice images may be accurately detected. The volumes of the specific parts may be accurately measured. Also, a plurality of 3-dimensional images of numerous parts of interest may be provided based on the contours of the parts of interest on the reference plane image and the slice images.

Although embodiments have been described with reference to a number of illustrative embodiments thereof, it should be understood that numerous other modifications and embodiments can be devised by those skilled in the art that will fall within the spirit and scope of the principles of this disclosure. More particularly, numerous variations and modifications are possible in the component parts and/or arrangements of the subject combination arrangement within the scope of the disclosure, the drawings and the appended claims. In addition to variations and modifications in the component parts and/or arrangements, alternative uses will also be apparent to those skilled in the art.

## Claims

1. An ultrasound system, comprising:
a volume data forming unit for forming volume data based on ultrasound echoes reflected from a target object, the target object including at least one part of interest;
an interface unit for allowing a user to input a selection instruction for selecting a reference plane from the volume data and a slice setting instruction for setting a plurality of slices perpendicular to the reference plane in the volume data;
an image processing unit for detecting a contour of the part of interest on the reference plane and determining intersection points of the detected contour and
each of the slices as seed points, the image processing unit being further configured to detect contours of the part of interest on the slices based on the seed points; and
a volume measuring unit for measuring a volume of the part of interest based on the contour on the reference plane and the contours on the slices.

2. The ultrasound system of Claim 1, wherein the image processing unit is further configured to form a 3-dimensional image of the part of interest based on the contour on the reference plane and the contours on the slices.

3. The ultrasound system of Claim 2, wherein the image processing unit includes:
a reference plane setting unit for setting the reference plane in the volume data in response to the selection instruction;
a contour setting unit for setting a contour of the part of interest on the reference plane image;
a slice setting unit for setting the slices in response to the slice setting instruction;
a seed point determining unit for determining intersection points of the contour set on the reference plane and each of the slices as the seed points; and
a contour detecting unit for detecting the contours of the part of interest on the slices based on the seed points.

4. The ultrasound system of Claim 3, wherein the volume measuring unit is configured to measure an area of the part of interest based on the contour set on the reference plane and an area of the part of interest based on the detected contour on each of the slices, the volume measuring unit being further configured to measure the volume of the part of interest based on the area of the part of interest based on the contour set on the reference plane and the area of the part of interest based on the detected contour on each of the slices.

5. The ultrasound system of Claim 3, wherein the seed point detecting unit detects two seed points and wherein the contour detecting unit sets a center point positioned at a center of said two seed points, the contour detecting unit being configured to detect edges in radial directions with respect to the center point and connect the detected edges to form the contour of the part of interest on each of the slices.

6. A method of measuring a volume of at least one part of interest contained in a target object in an ultrasound system including a volume data forming unit, an interface unit, an image processing unit and a volume measuring unit, the method comprising:
a) forming volume data based on ultrasound echoes reflected from a target object containing at least one part of interest at the volume data forming
unit;
b) receiving a selection instruction for selecting a reference plane from the volume data at the interface unit;
c) detecting a contour of the part of interest on the reference plane at the image processing unit;
d) receiving a slice setting instruction for setting a plurality of slices perpendicular to the reference plane in the volume data at the interface
unit;
e) determining intersection points of the detected contour and each of the slices as seed points and detecting contours of the part of interest on the slices based on the seed points at the image processing unit; and
f) measuring a volume of the part of interest based on the contour on the reference plane and the contours on the slices at the volume measuring unit.

7. The method of Claim 6, further comprising forming a 3-dimensional image of the part of interest based on the contour on the reference plane and the contours on the slices at the image processing unit.

8. The method of Claim 6, wherein the step e) includes:
detecting two intersection points of the contour of the part of interest on the reference plane and each of the slices;
setting a center point of two intersection points;
detecting edges of the part of interest in radial directions with respect to the center point; and
connecting the edges to detect the contour of the part of interest.

9. The method of Claim 6, wherein the step f) includes:
measuring an area of the part of interest based on the contour set on the reference plane and an area of the part of interest based on the detected contour on each of the slices; and
measuring the volume of the part of interest based on the area of the part of interest based on the contour set on the reference plane and the area of the part of interest based on the detected contour on each of the slices.
